# EUROPÄISCHE PATENTSCHRIFT

(11) **EP 1 383 872 B1**
(45) Veröffentlichungstag und Bekanntmachung des Hinweises auf die Patenterteilung: **23.11.2005**
(21) Anmeldenummer: 02742682.4
(22) Anmeldetag: 18.04.2002
(51) Int. Cl.: C12N 5/10, C12Q 1/02, A61P 35/00

(54) **GENMODIFIZIERTE YT ZELLLINIE UND IHRE VERWENDUNG**
GENETICALLY MODIFIED YT CELL LINE AND USE THEREOF
LIGNEE CELLULAIRE YT GENETIQUEMENT MODIFIEE ET SON UTILISATION

(30) Priorität: 28.04.2001 DE 10121113
(43) Veröffentlichungstag der Anmeldung: 28.01.2004
(73) Patentinhaber: Pecher, Gabriele, 10179 Berlin (DE)
(72) Erfinder: Pecher, Gabriele, 10179 Berlin (DE)
(74) Vertreter: Baumbach, Friedrich
(86) Internationale Anmeldenummer: PCT/DE2002/001437
(87) Internationale Veröffentlichungsnummer: WO 2002/088336

(56) Entgegenhaltungen:
- WO-A-00/23573
- WO-A-95/30014
- WO-A-97/15669
- YODOI J ET AL: "T CELL GROWTH FACTOR INTERLEUKIN 2-RECEPTOR-INDUCING FACTORS 1. REGULATION OF INTERLEUKIN 2 RECEPTOR ON A NATURAL KILLER-LIKE CELL LINE YT CELLS" JOURNAL OF IMMUNOLOGY, Bd. 134, Nr. 3, 1985, Seiten 1623-1630, XP002226418 ISSN: 0022-1767 in der Anmeldung erwähnt
- KLINGEMANN H-G ET AL: "DEVELOPMENT OF NK-92 CELLS FOR IMMUNOTHERAPY OF MALIGNANCIES" JOURNAL OF IMMUNOTHERAPY, RAVEN PRESS, NEW YORK, US, Bd. 23, Nr. 5, September 2000 (2000-09), Seite 604 XP008009924 ISSN: 1053-8550
- SCHIRRMANN THOMAS ET AL: "Tumor-specific targeting of a cell line with natural killer cell activity by asialoglycoprotein receptor gene transfer." CANCER IMMUNOLOGY IMMUNOTHERAPY, Bd. 50, Nr. 10, Dezember 2001 (2001-12), Seiten 549-556, XP002226419 ISSN: 0340-7004

## Beschreibung

Die Erfindung betrifft eine gentechnisch veränderte YT-Zelllinie, in die die Gene von Rezeptoren gebracht wurden und die z. B. zur Behandlung und zur Diagnostik von Tumoren, Tumormetastasen, Autoimmunerkrankungen, Virusinfektionen und zur Reinigung (Purging) von Blutstammzellpräparaten geeignet ist. Anwendungsgebiete sind die Medizin und die pharmazeutische Industrie.

Die nicht genetisch veränderte Zelllinie YT wurde erstmals beschrieben von Yodoi et al. (Yodoi-J; Teshigawara-K; Nikaido-T; Fukui-K; Noma-T; Honjo-T; Takigawa-M; Sasaki-M; Minato-N; Tsudo-M; et-al. TCGF (IL 2)-receptor inducing factor(s). I. Regulation of IL 2 receptor on a natural killer-like cell line (YT cells). J-Immunol. 1985 Mar; 134(3): 1623-30). Sie wurde aus dem Blut eines Patienten etabliert. Diese Zellinie ist per se nicht in der Lage, Tumore oder andere Ziellzellen spezifisch zu erkennen und spezifisch zu lysieren.
In der Literatur sind außerdem unterschiedliche Systeme mit chimären Genen beziehungsweise Proteinen beschrieben worden.
Groner et al (WO 95 30014 A) beschreibt ein bifunktionelles Protein, jedoch keine (immortalisierte) Zelllinie. Ein DNA-Konstrukt, das für dieses Protein kodiert, kann Groners Beschreibung nach in T-Zellen (= T-Lymphozyten) eines Spenders gebracht werden. T-Zellen sind jedoch keine NK-Zellen, da sie z.B. im Gegensatz zu NK-Zellen von Hause aus bereits über einen eigenen Rezeptor verfügen, sie haben daher andere Wirk- und Erkennungsmechanismen.
Auch in der analogen Publikation, Rosenberg et al (WO 97 15669 A) wird keine immortalisierte NK-Zelllinie beschrieben, in die Gene verbracht werden.
Bei Raubitschek et al (1998, WO 00 23573 A) handelt sich ebenfalls nur um T-Zellen, nicht um Zelllinien und/oder NK-Zellen. Darüber hinaus werden T-Zellen beschrieben, die ein Zelloberflächenprotein exprimieren, das spezifisch für CD20 ist und außerdem eine intrazelluläre Signaldomäne und eine Transmembrandomäne aufweist. Diese T-Zellen werden nur gegen das Molekül CD20 gerichtet.

Die Erfindung hat das Ziel, das Erkennen von Tumorzellen und anderen Zielzellen über den Gentransfer von spezifischen Rezeptoren in die YT-Zelllinie, zu erreichen. Eine weitere Aufgabe besteht darin, mit der neu geschaffenen Zelllinie eine Lyse der erkannten Zielzellen zu ermöglichen.

Die Erfindung wird gemäß den Patentansprüchen realisiert. Kernpunkt der Erfindung ist die immortalisierte NK-Zelllinie YT, die durch Gentransfer von Fremdgenen modifiziert ist und zur Therapie und zur Diagnose von menschlichen und tierischen Erkrankungen verwendbar ist. Erfindungsgemäß werden dazu Fremdgene eingebracht, bei denen es sich um ein oder mehrere Rezeptorgene handelt, die für Rezeptoren kodieren, die die jeweiligen Zielzellen spezifisch erkennen, sowie ggf. um weitere Gene.
In einer vorteilhaften Ausführung der Erfindung enthält der Rezeptor eine Erkennungsdomäne, welche Strukturen auf den jeweiligen Zielzellen bzw. Tumorzellen oder virusinfizierten Zellen oder auch autoimmunreaktiven Zellen spezifisch erkennt. Das können Domänen sein, die aus variablen Regionen tumor- oder Zielzellen-spezifischer Antikörper (single chain Fv- Fragmente) bestehen.
In vielen Fällen enthält der eingebrachte Rezeptor zusätzlich eine Signalkette, die in der Lage ist, ein Signal zur Auslösung zytolytischer Aktivität der Zelllinie YT zu vermitteln.
Die Signalkette des eingebrachten Rezeptors kann von der zeta-Kette des humanen T-Zellrezeptors abgeleitet sein. Sie kann aber auch über eine Verbindungsdomäne mit der Erkennungsdomäne verbunden sein. Die Verbindungsdomäne ist in aller Regel von dem humanen IgG-Fc-Teil abgeleitet.
In einer speziellen Ausführungsform sind Rezeptordomänen spezifisch für die Humanen Tumorantigene Carcinoembryonales Antigen (CEA), Muzin oder CD33.
Die weitere Proliferation der Zelllinie wird vermieden, wenn die Zelllinie gamma-bestrahlt oder durch mehrere, beispielsweise durch Elektroporation, eingebrachte Suizidgene gegenüber bestimmten Medikamenten bzw. Chemotherapeutika bzw. Substanzen sensibel gemacht wird.
Die Zelllinie kann mit einem Farb- oder radioaktiven Stoff markiert werden, wodurch ein schneller Nachweis gewährleistet ist. Die Zelllinie wird gentechnisch verändert und kann dadurch in der Diagnose und in der Therapie verwendet.werden. Erfindungsgemäß werden Gene für Rezeptoren, die spezifisch Tumorzellen oder andere Zielzellen erkennen, per Gentransfer in die YT-Zelllinie eingebracht. Diese Rezeptorkonstrukte enthalten eine aus dem erkennenden Teil eines Antikörpers hergestellte Domäne, die Tumorzellen oder andere Zielzellen bzw. deren Strukturen spezifisch erkennt. Mit dieser Domäne ist eine Signalkette, abgeleitet aus der zeta-Kette des humanen T-Zellrezeptors, verbunden, die die Lyseaktivität vermittelt.

Diese Merkmale werden nachfolgend noch einmal zusammengefasst.

Die Vorteile der Erfindung bestehen im folgenden:
- Die YT- Zellinie eignet sich für den Gentransfer von Rezeptoren und erhält durch den Rezeptorgentransfer neue Eigenschaften, die z. B. in der Erkennung und Lyse von Tumorzellen und anderen Zielzellen bestehen.
- Die neue Zelllinie ist immortalisiert und deshalb unbegrenzt verfügbar.
- Die Zelllinie läßt sich durch entsprechende Wahl der Rezeptoren gegen verschiedene Tumorzellen oder andere Zielzellen abrichten.
- Sie verliert auch durch gamma-Bestrahlung ihre Wirksamkeit nicht. Durch Bestrahlung wird ihre weitere Proliferation verhindert, die Killereigenschaften bleiben jedoch erhalten, so daß 1-2 Tage noch mit einer ausreichenden Rezeptorwirkung gerechnet werden kann.

Die Erfindung soll nachfolgend durch Ausführungsbeispiele näher erläutert werden.

### Beispiel

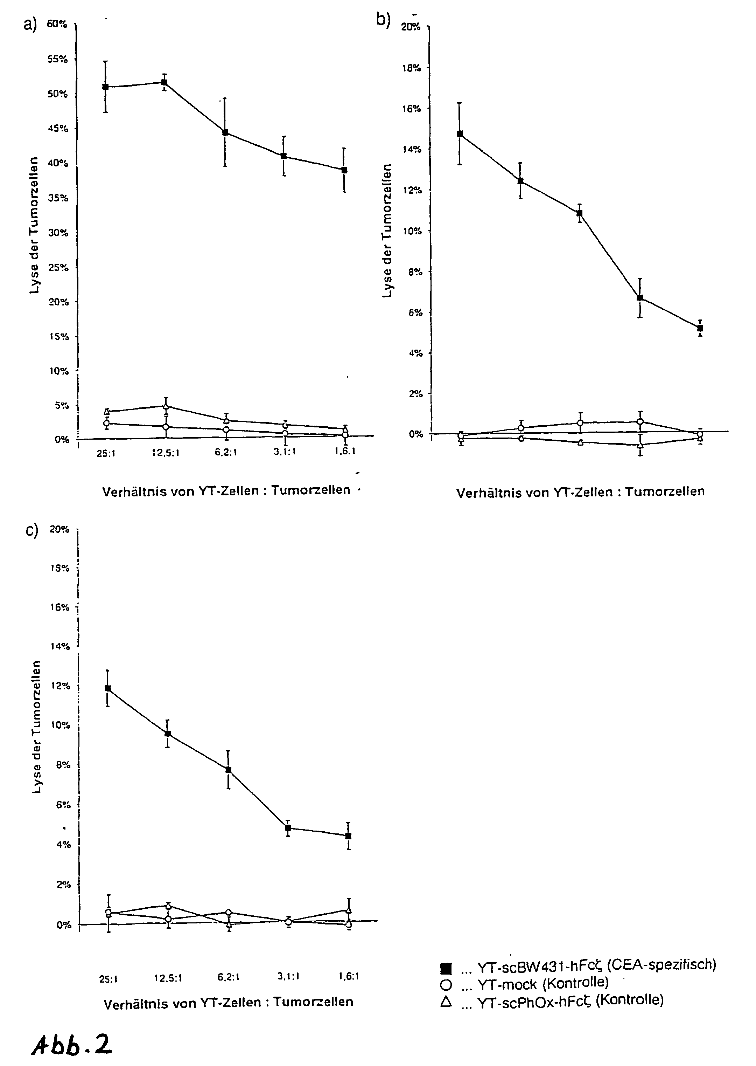

Es wurde ein Rezeptor konstruiert, der spezifisch die Struktur Carcinoembryonales Antigen (CEA) auf Darmkrebszellen erkennt. Die Gene dieses Rezeptors wurden durch Elektoporation als Gentransferverfahren in die YT-Zelllinie gebracht.

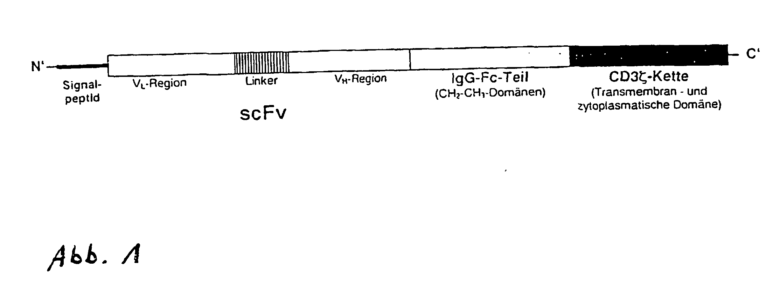

Abb. 1 zeigt die Zusammensetzung des Rezeptors, der per Gentransfer in die YT-Zellinie gebracht wurde. Der Rezeptor besteht in seinen wesentlichen Bestandteilen aus einem spezifischen single-chain Fv (scFv) Fragment, das aus den variablen Regionen des humanisierten Antikörpers BW431, der spezifisch Strukturen des CEA erkennt, hergestellt ist. Dieses Fragment ist über einen Peptidlinker mit der zeta-(ζ)-Kette des humanen T-Zellrezeptors, die die Lyseaktivität vermittelt, verbunden.
Die mit diesem Rezeptor per Gentransfer ausgestattete YT-Zellinie wurde dann auf ihre Lyse (Killer)-aktivität von Darm (Kolon)-Tumorzellen hin getestet. Abb. 2 zeigt, dass die Rezeptorgentransfizierte YT-Zelllinie die humanen Kolonkarzinomzellinien LS174T und SW1222 sowie die Zellinie MC32A, die humanes CEA auf der Oberfäche hat, lysiert. Kontroll-YT-Zellen, in die das genannten spezifischen Rezeptorkonstrukt nicht hineingebracht wurde, sondern andere nicht relevante Gene (mock, scPhOx-hFcζ) waren nicht in der Lage, die Tumorzellen in einem nach üblichen Standardbedingungen ausgeführten ⁵¹Cr-Freisetzungsassay signifikant zu lysieren.

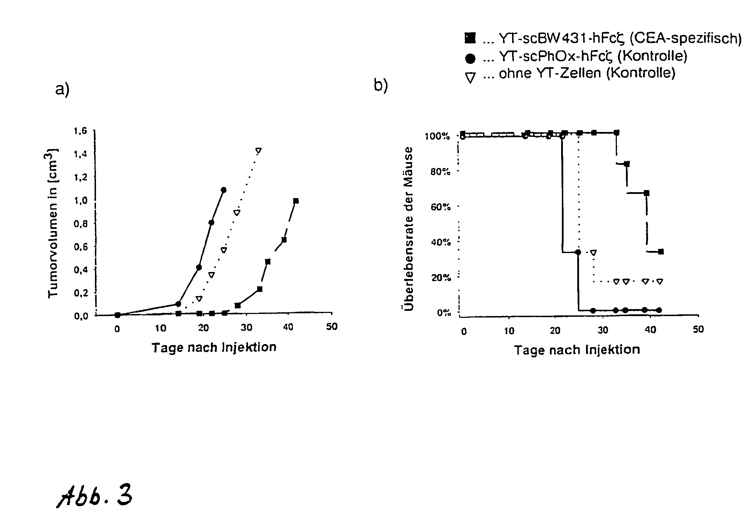

Weiterhin wurde die Effektivität der Lyse von Tumoren durch die CEA-Rezeptorgentranfer modifizierte und in ihrer Proliferation durch Gamma-Bestrahlung gehemmte YT-Zelllinie in einem Maus-Tumor-Modell überprüft. Abb. 3 zeigt, dass wenn die CEA tragenden Tumorzellen MC32A zusammen mit der CEA-erkennenden genmodfizierten YT-Zelllinie den Mäusen appliziert wurden, ein Tumorwachstum verzögert wurde. Die Mäuse überlebten länger im Vergleich dazu, wenn eine mit nicht relevanten Genen als Kontrolle transfizierte YT-Zellinie zusammen mit den Tumorzellen appliziert wurde.

Die so genetisch veränderte YT-Zelllinie kann für die Therapie von menschlichen CEAtragenden Tumoren angewendet angewendet werden, indem sie direkt in den Tumor oder in vorhandene Metastasen oder systemisch intravenös appliziert wird. Die genetisch veränderte YT-Zelllinie erkennt das CEA auf den Tumoren und lysiert diese Tumorzellen. Die Lyse wird vermittelt durch die in den Rezeptor "eingebaute" Signalkette. Die Zelllinie kann auch benutzt werden, um in Stammzellpräparaten oder anderen Präparaten Tumorzellen zu erkennen und zu lysieren, oder in der Diagnostik, wenn man die erfindungsgemäßen Zellen entsprechend markiert (z. B. mit einem Farbstoff oder einem radioaktiven Stoff), zum Auffinden von Tumorzellen und Metastasen nach z. B. intravenöser Applikation.

### Legenden zu den Abbildungen:

### Abbildung 1 - Schema der spezifischen Fusionsrezeptoren

Die Rezeptoren werden N'-terminal durch ein Signalpeptid, das für die Expression der Rezeptoren auf der Oberfläche der YT-Zellen notwendig ist, eingeleitet. Die Rezeptoren bestehen aus einem spezifischen single-chain-Fv- (scFv-)Fragment, das aus den variablen Regionen eines Antikörpers (im Beispiel vom humanisierten CEA-spezifischen Antikörper BW431) durch Verknüpfung über einen flexiblen Peptidlinker konstruiert ist, einem Distanzhalter zwischen scFv-Fragment und Zellmembran (im Beispiel der Fc-Teil vom humanen Antikörper (IgG-Fc)) und dem signalauslösenden Teil der ζ-Kette des humanen T-Zellrezeptorkomplexes.

### Abbildung 2- ⁵¹Cr-Freisetzungsassay zur Bestimmung der Lyse von CEAexprimierenden Tumorzelllinien durch YT-Zellen, in die durch Gentransfer der CEA-spezifische Fusionsrezeptor scBW431-hFcζ hineingebracht wurde

Die YT-Zellinie wurde durch Gentransfer mit dem Rezeptor scBW431-hFcζ, der spezifisch für humanes CEA ist, modifiziert. Die Zytotoxizität dieser YT-Zellen wurden gegenüber der Zelllinie a) MC32A, die humanes CEA auf der Oberfläche hat, und den humanen Kolonkarzinomzelllinien b) LS174T und c) SW 1222 getestet.
Als Kontrollen wurden auch YT-Zellen eingesetzt, die entweder nur mit dem Leervektor (*mock*) oder mit dem Fusionsrezeptor scPhOxhFcζ, der keine Struktur auf den Tumorzellen erkennen kann, transfiziert.

### Abbildung 3 - Nod/SCID-Maus-Experiment

Am Tag 0 wurden Nod/SCID-Mäusen MC32A-Tumorzellen zusammen mit CEA-spezifischen YT-Zellen (YT-scBW431-hFcζ) oder phOx-spezifischen YT-Zellen (YT-scPhOx-hFcζ) oder ohne YT-Zellen unter die Haut injiziert. Die eingesetzten YT-Zellen wurden zuvor durch γ-Bestrahlung in ihrem weiteren Wachstum gehemmt. In der Abbildung wurden a) das Tumorwachstum und b) die Überlebensrate nach Injektion der Zellen angegeben. Tiere mit einem Tumor größer als 1cm³ wurden It. Tierschutzrichtlinien getötet und entsprechend als tot eingestuft.

## Patentansprüche

1. YT-Zelllinie, die durch Gentransfer modifiziert ist und zur Therapie und zur Diagnose von menschlichen und tierischen Erkrankungen verwendet wird, **dadurch gekennzeichnet, dass** Fremdgene eingebracht sind, wobei es sich um ein oder mehrere Rezeptorgene handelt, die die jeweiligen Zielzellen spezifisch erkennen, sowie ggf. um weitere Gene.

2. YT-Zelllinie nach Anspruch 1, **dadurch gekennzeichnet, dass** der Rezeptor eine Erkennungs-Domäne enthält, die Strukturen auf den jeweiligen Zielzellen spezifisch erkennt.

3. YT-Zelllinie nach Anspruch 1, **dadurch gekennzeichnet, dass** der Rezeptor eine Domäne enthält, die Strukturen auf Tumorzellen spezifisch erkennt.

4. YT-Zelllinie nach Anspruch 1, **dadurch gekennzeichnet, dass** der Rezeptor eine Domäne enthält, die Strukturen auf virusinfizierten Zellen spezifisch erkennt.

5. YT-Zelllinie nach Anspruch 1, **dadurch gekennzeichnet, dass** der Rezeptor eine Domäne enthält, die Strukturen auf autoimmunreaktiven Immunzellen spezifisch erkennt.

6. Zelllinie nach Anspruch 1 **dadurch gekennzeichnet, dass** die Rezeptordomäne aus variablen Regionen tumor- oder Zielzellen-spezifischer Antikörper (single chain Fv-Fragmente) besteht.

7. Zelllinie nach Anspruch 1, **dadurch gekennzeichnet, dass** der eingebrachte Rezeptor zusätzlich eine Signalkette, die in der Lage ist, ein Signal zur Auslösung der zytolytischer Aktivität der Zelllinie YT zu vermitteln, enthält.

8. Zelllinie nach Anspruch 1, **dadurch gekennzeichnet, dass** die Signalkette des eingebrachten Rezeptors von der zeta-Kette des humanen T-Zellrezeptors abgeleitet ist.

9. Zelllinie nach Anspruch 1, **dadurch gekennzeichnet, dass** die Signalkette des eingebrachten Rezeptors über eine Verbindungsdomäne mit der Erkennungsdomäne verbunden ist.

10. Zelllinie nach Anspruch 1, **dadurch gekennzeichnet, dass** die Verbindungsdomäne von dem humanen IgG-Fc-Teil abgeleitet ist.

11. Zelllinie nach Anspruch 1, **dadurch gekennzeichnet, dass** die Rezeptordomänen spezifisch an die humanen Tumorantigene Carcinoembryonales Antigen CEA, Muzin oder CD₃₃ binden

12. Zelllinie nach Anspruch 1, **dadurch gekennzeichnet dass** sie zur Vermeidung weiterer Proliferation gamma-bestrahlt wird.

13. Zellinie nach Anspruch 1, **dadurch gekennzeichnet, dass** in diese Zelllinie zusätzlich ein oder mehrere Suizidgene eingebracht werden, durch die die Zelllinie gegenüber bestimmten Medikamenten/Chemotherapeutika/Substanzen sensibel gemacht wird.

14. Zelllinie nach Anspruch 1, **dadurch gekennzeichnet, dass** die Gene für das Rezeptorkonstrukt bzw. die Rezeptorkonstrukte und ggf. das Suizidgen bzw. die Suizidgene durch Elektroporation in die Zellinie eingebracht sind.

15. Zelllinie nach Anspruch 1, **dadurch gekennzeichnet, dass** sie mit einem Farb- oder radioaktiven Stoff markiert ist.

## Claims

1. YT cell line, which is modified by means of gene transfer and can be used in the therapy and diagnostics of human and animal diseases, **characterized by** the fact that foreign genes are integrated encoding one or several receptors which specifically recognise the respective target cells, and, if necessary, additional genes.

2. YT cell line in accordance with Claim 1, **characterized by** the fact that the receptor contains a recognition domain specifically recognising structures on the respective target cells.

3. YT cell line in accordance with Claim 1, **characterized by** the fact that the receptor contains a recognition domain specifically recognising structures on tumour cells.

4. YT cell line in accordance with Claim 1, **characterized by** the fact that the receptor contains a recognition domain specifically recognising structures on virus-infected cells.

5. YT cell line in accordance with Claim 1, **characterized by** the fact that the receptor contains a recognition domain specifically recognising structures on autoimmune-reactive immune cells.

6. Cell line in accordance with Claim 1, **characterized by** the fact that the receptor domain consists of variable regions of antibodies specific of the tumour or target cells (single chain Fv fragments).

7. Cell line in accordance with Claim 1, **characterized by** the fact that the incorporated receptor additionally contains a signal chain which is capable of mediating a signal to induce cytolytic activity of the cell line YT.

8. Cell line, **characterized by** the fact that the signal chain of the incorporated receptor is derived from the zeta-chain of the human T cell receptor.

9. Cell line, **characterized by** the fact that the signal chain of the incorporated receptor is coupled to the recognition domain by means of a linker domain.

10. Cell line, **characterized by** the fact that the linker domain is derived from the human IgG-Fc part.

11. Cell line in accordance with Claim 1, **characterized by** the fact that the receptor domains bind specifically to the human tumour antigens Carcinoembryonic Antigen (CEA), mucin, or CD33.

12. Cell line in accordance with Claim 1, **characterized by** the fact that the cell line is exposed to gamma radiation to prevent further proliferation.

13. Cell line in accordance with Claim 1, **characterized by** the fact that one or several suicide genes are additionally incorporated into this cell line that makes it susceptible to the action of certain pharmaceuticals/chemotherapeutics/substances.

14. Cell line in accordance with Claim 1, **characterized by** the fact that the genes encoding the receptor construct or receptor constructs, and, if necessary, the suicide gene or suicide genes, are incorporated into the cell line by means of electroporation.

15. Cell line in accordance with Claim 1, **characterized by** the fact that the cell line is labelled with a dye or radioactive substance.

## Revendications

1. Ligne cellulaire YT qui est modifiée par transfert génétique et utilisée pour le traitement et le diagnostic de maladies humaines et animales, **se caractérisant par le fait que** des gènes étrangers y ont été intégrés, s'agissant ici d'un gène récepteur ou de plusieurs gènes récepteurs qui reconnaissent de façon spécifique les cellules cibles en question, et s'agissant aussi le cas échéant d'autres gènes.

2. Ligne cellulaire YT selon la revendication 1, **se caractérisant par le fait que** le récepteur contient un domaine de reconnaissance qui reconnaît de façon spécifique les structures sur les cellules cibles en question.

3. Ligne cellulaire YT selon la revendication 1, **se caractérisant par le fait que** le récepteur contient un domaine qui reconnaît de façon spécifique les structures sur des cellules tumorales.

4. Ligne cellulaire YT selon la revendication 1, **se caractérisant par le fait que** le récepteur contient un domaine qui reconnaît de façon spécifique les structures sur des cellules infectées par un virus.

5. Ligne cellulaire YT selon la revendication 1, **se caractérisant par le fait que** le récepteur contient un domaine qui reconnaît de façon spécifique les structures sur des cellules immunitaires à réaction auto-immunitaire.

6. Ligne cellulaire selon la revendication 1, **se caractérisant par le fait que** le domaine de récepteur se compose d'anticorps spécifiques à la tumeur ou spécifiques aux cellules cibles (fragments Fv single chain) en provenance de régions variables.

7. Ligne cellulaire selon la revendication 1, **se caractérisant par le fait que** le récepteur inséré contient en plus une chaîne signal qui est en mesure de communiquer un signal pour déclencher l'activité cytolytique de la ligne cellulaire YT.

8. Ligne cellulaire selon la revendication 1, **se caractérisant par le fait que** la chaîne signal du récepteur inséré dérive de la chaîne zêta du récepteur humain de la cellule T.

9. Ligne cellulaire selon la revendication 1, **se caractérisant par le fait que** la chaîne signal du récepteur inséré est reliée au domaine de reconnaissance via un domaine de connexion.

10. Ligne cellulaire selon la revendication 1, **se caractérisant par le fait que** le domaine de connexion dérive de la partie IgG-Fc humaine.

11. Ligne cellulaire selon la revendication 1, **se caractérisant par le fait que** les domaines de récepteur relient de façon spécifique l'antigène carcino-embryonnaire CEA, la mucine ou le CD33 aux antigènes tumoraux humains.

12. Ligne cellulaire selon la revendication 1, **se caractérisant par le fait qu'**elle est irradiée gamma pour éviter d'autre prolifération.

13. Ligne cellulaire selon la revendication 1, **se caractérisant par le fait qu'**un ou plusieurs gènes suicides sont insérés en plus dans cette ligne cellulaire, gènes qui sensibilisent la ligne cellulaire à certains médicaments/médicaments chimiothérapeutiques/substances.

14. Ligne cellulaire selon la revendication 1, **se caractérisant par le fait que** les gènes pour la construction du récepteur voire les constructions de récepteur et le cas échéant le gène suicide voire les gènes suicides sont insérés par électroporation dans la ligne cellulaire.

15. Ligne cellulaire selon la revendication 1, **se caractérisant par le fait qu'**elle est marquée avec un colorant ou une substance radioactive.
